# EUROPÄISCHE PATENTSCHRIFT

(11) **EP 3 368 117 B1**
(45) Veröffentlichungstag und Bekanntmachung des Hinweises auf die Patenterteilung: **14.08.2024**
(21) Anmeldenummer: 15801113.0
(22) Anmeldetag: 28.10.2015
(51) Int. Cl.: A61M 25/00, A61M 25/01, A61B 17/22

(54) **ASPIRATIONSKATHETER**
ASPIRATION CATHETER
CATHÉTER D'ASPIRATION

(43) Veröffentlichungstag der Anmeldung: 05.09.2018
(73) Patentinhaber: STG Medical AG, 8404 Winterthur (CH)
(72) Erfinder: SCHWAGER, Michael, 8404 Winterthur (CH)
(74) Vertreter: Keller Schneider Patent- und Markenanwälte AG
(86) Internationale Anmeldenummer: PCT/CH2015/000157
(87) Internationale Veröffentlichungsnummer: WO 2017/070801

(56) Entgegenhaltungen:
- EP-A1- 1 820 531
- WO-A2-99/56801
- DE-A1- 102011 111 534
- JP-A- 2006 280 636
- JP-A- 2006 280 668
- US-A- 5 916 192
- US-A1- 2007 197 956

## Beschreibung

### Technisches Gebiet

Die vorliegende Erfindung betrifft einen Aspirationskatheter zur Aspiration von Material aus Blutgefässen. Weiter betrifft die Erfindung einen Komponentensatz zur Aspiration von Material aus Blutgefässen sowie entsprechende Verfahren.

Weitere Aspekte der Erfindung befassen sich mit der Anordnung radioopaker Markierungen an Aspirationskathetern, sowie der Farbkodierung von Komponentensätzen zur Aspiration von Material aus Blutgefässen.

### Stand der Technik

Ansammlungen von körpereigenem oder fremdem Material können in einem Blutgefäss zu einem teilweisen oder vollständigen Verschluss des betroffenen Blutgefässes führen. Durch solche Verstopfungen kann die Blutversorgung von Geweben und Organen bis zum vollständigen Ausfall beeinträchtigt werden. Beispiele für solche Krankheitsbilder sind Infarkte, Thrombosen und Embolien. Bei einem Thrombus entsteht ein Pfropfen aus koaguliertem Blut, während es sich beim Embolus um einen Klumpen im Blutstrom vorhandenen Materials, wie zum Beispiel Fetten bei Fettembolien oder Eiter bei septischen Embolien, die zusätzlich bakteriell infiziert sind, handelt.

Solche Verstopfungen können sowohl arteriell als auch venös vorkommen. Eine besondere Form der arteriellen Embolie kann den Infarkt des betroffenen unterversorgten Gewebes verursachen, was wiederum zu einem Herzinfarkt oder Hirnschlag führen kann. Neben anderen im Akutfall angewandten medizinischen Verfahren, die zur Lebenserhaltung oder Notfallversorgung des betroffenen Patienten dienen, ist es üblich, das verstopfende Material zu entfernen und/oder die betroffene Gefässwand wieder zu dehnen. Mittels Kathetern werden die Verstopfungen beseitigt, d.h. abgesogen und anschliessend mit Stents und/oder Ballonen das betroffene Gewebe gestützt oder geweitet werden. Solche Eingriffe finden perkutan statt und sind Routineprozesse, insbesondere bei koronaren Interventionen.

Für die Aspiration von Thromben und/oder Embolien aus Blutgefässen werden speziell angepasste Katheter eingesetzt. Solche Katheter weisen neben einem für die Durchfuhr durch die Blutgefässe geeigneten Durchmesser ein Aspirationslumen auf, durch welches bei Anbringen einer Saugwirkung an einer Seite durch eine am anderen Ende vorgesehene Aspirationsöffnung aus Blutgefässen abgesaugt werden kann. Neben diesem ersten Lumen verfügen Aspirationskatheter zudem über mindestens ein zweites Lumen, welches dazu dient, einen Führungsdraht aufzunehmen. Dieser Führungsdraht dient dazu, den Katheter in den Blutgefässen zu führen und am betreffenden Absaugort zu platzieren. Eine besondere Form von Kathetern sind Katheter des sogenannten "Rapid Exchange"-Typs. Bei diesen Kathetern wird der Führungsdraht nur durch ein vergleichsweise kürzeres Segment des Katheters durchgeführt. Mit solchen Kathetern kann der behandelnde Chirurg die Katheter schneller und einfacher auswechseln. Nur im geführten Bereich des Katheters verläuft ein Führungsdrahtlumen, anschliessend tritt es lateral am Katheter aus und der Führungsdraht verläuft im Gefässsystem weitgehend parallel und benachbart zum Katheter.

Für das Absaugen des Materials wird der Katheter durch das betroffene Blutgefäss bis zur obstruierten Stelle vorgeschoben. Dabei können radioopake Markierungen am Katheter die exakte Lokalisation und Platzierung ermöglichen. Erreicht der Katheter den Obstruktionsort, so wird er durch den Pfropfen, respektive Embolus hindurchgestossen. Das eigentliche Absaugen findet dann unter Rückzug des Katheters statt, und zwar von proximal zu distal. Wird die Aspirationsöffnung durch das abzusaugende Material gezogen, so wird dieses aspiriert. Um ein gleichmässiges Aspirieren zu fördern, kann der Katheter zudem um die eigene Längsachse gedreht werden. Drehungen des Katheters sind zuweilen auch bei der Platzierung erforderlich.

Der Katheter muss von der perkutanen Öffnung bis zum Obstruktionsort vorgeschoben werden. Entscheidend für die Platzierung und die Sicherheit des Katheters sind sowohl seine Flexibilität als auch seine Steifigkeit. Bei konventionellen Kathetern ist der mindestens eine Bereich, in dem das Führungsdrahtlumen untergebracht ist, für eine lateral einseitige Versteifung des Katheters verantwortlich. Beim Drehen des Katheters, sowohl während der Platzierung, als auch beim Absaugprozess, gerät der Katheter in eine Schlagbewegung. Das heisst die Rotation der Spitze um die eigene Achse ist durch vorangegangene Wölbungen des Katheters erschwert oder behindert, und zwar dergestalt, dass keine gleichmässige Rotation mehr möglich ist, sondern ein "Schlagen" des Katheters stattfindet. Dies senkt nicht nur die Effektivität des Absaugprozesses, sondern führt auch zusätzlich zur Irritation der Gefässinnenwand.

Die EP 1011775 B1 (Bagaoisan, C. J., et al.) beschreibt einen Aspirationskatheter, welcher geeignet ist, Plaquethromben oder andere Materialien aus Gefässen abzusaugen. Der gezeigte Katheter besteht aus einem langen, schlauchförmigen Körper mit einem proximalen und einem distalen Ende. Zwischen diesen erstreckt sich ein Aspirationslumen mit einer Aspirationsöffnung am distalen Ende. Bei diesem gezeigten Katheter handelt es sich um einen "Rapid Exchange"-Katheter, bei dem weniger als 40 cm des distalen Bereichs in proximaler Richtung ein Führungsdrahtlumen aufweist. Am proximalen Ende wird eine Spritze angebracht, welche mittels einer Unterdruckerzeugung einen Absaugeffekt bewirkt. Führungsdrahtlumen und Aspirationslumen verlaufen parallel und sind vollständig getrennt. Aus der Geometrie des hier gezeigten Katheters geht aber klar hervor, dass dieser Katheter nicht geeignet ist, um verdreht um die eigene Achse zu rotieren. Das aussen entlang des Aspirationslumens parallel verlaufende Führungsdrahtlumen behindert eine freie Rotation und sorgt für Schlag- und Twist-Bewegungen.

Auch die WO 2013/029795 A1 (Gülcher, M., et al.) zeigt einen Aspirationskatheter des "Rapid Exchange"-Typs. Dieser Katheter dient dazu, thrombotisches Material aus Blutgefässen abzusaugen und verfügt über ein erstes und ein zweites Lumen. Das erste Lumen bildet das Aspirationslumen, während das zweite Lumen koaxial zum ersten Lumen verläuft und ein Führungsdrahtlumen bildet. Dieses Führungsdrahtlumen ist allerdings an der Katheterinnenwand festgelegt. Dadurch resultiert eine einseitige Steifigkeit des Katheters, und es tritt eine entsprechende Behinderung der Rotation um die eigene Achse ein.

Siehe auch EP1820531 A1 und JP2006280668 A.

Somit besteht ein Bedürfnis nach einem Aspirationskatheter, der einfach zu bedienen ist und mindestens einen Nachteil des Bekannten überwindet.

### Darstellung der Erfindung

Die Aufgabe der vorliegenden Erfindung ist es somit, einen Aspirationskatheter bereitzustellen, der mindestens einen Nachteil des Bekannten überwindet. Insbesondere soll ein Aspirationskatheter, ein Komponentensatz zur Aspiration von Material aus Blutgefässen, sowie ein Verfahren zu dessen Bedienung bereitgestellt werden, welches einfach und effizient herzustellen ist und bevorzugt über ein geringeres Schlagen und/oder Twisten bei der Rotation um die eigene Achse zeigt.

Diese Aufgabe wurde mit einem Aspirationskatheter gemäß dem unabhängigen Anspruch 1 der vorliegenden Erfindung gelöst.

Ein Aspekt der vorliegenden Erfindung betrifft einen Aspirationskatheter zur Aspiration von Material aus Blutgefässen. Der erfindungsgemässe Aspirationskatheter umfasst eine röhrenförmige Katheterwandung mit einer ersten distalen Öffnung und einer ersten proximalen Öffnung. Diese beiden Öffnungen sind durch ein erstes Lumen verbunden. Der Aspirationskatheter weist zudem einen röhrenförmigen Führungsmantel mit einer zweiten distalen Öffnung und einer zweiten proximalen Öffnung auf. Auch die zweite distale Öffnung und die zweite proximale Öffnung sind durch ein zweites Lumen verbunden. Der röhrenförmige Führungsmantel ist innerhalb des ersten Lumens angeordnet. Das zweite Lumen verläuft koaxial zum ersten Lumen. Die zweite proximale Öffnung des röhrenförmigen Führungsmantels erstreckt sich lateral durch die röhrenförmige Katheterwandung. Der röhrenförmige Führungsmantel ist in einem Bereich der ersten distalen Öffnung und in einem Bereich der zweiten proximalen Öffnung mit der röhrenförmigen Katheterwandung verbunden.

Im Sinne der vorliegenden Erfindung sind die Begriffe proximal und distal ausgehend von der Körpermitte des einen erfindungsgemässen Aspirationskatheter benutzenden Arztes zu betrachten.

Im Sinne der vorliegenden Erfindung ist ein Lumen eine Ausnehmung, in welcher eine vollständige Fluidverbindung besteht. Eine Öffnung in einem solchen Lumen kann dabei einen Fluidkontakt mit einem weiteren Lumen, zum Beispiel dem Innenraum eines Blutgefässes oder eines Absauggerätes, ermöglichen.

In einer besonderen Ausführungsform bedeutet röhrenförmig einen im Wesentlichen runden Querschnitt. Ein Querschnitt ist im Wesentlichen rund, wenn er um nicht mehr als 10 % von einer Kreisform abweicht.

Bei der vorliegenden Erfindung ist der röhrenförmige Führungsmantel im Bereich der zweiten distalen Öffnung und im Bereich der zweiten proximalen Öffnung mit der röhrenförmigen Katheterwandung stoffschlüssig verbunden. Das heisst die beiden Elemente, die röhrenförmige Katheterwandung und der röhrenförmige Führungsmantel, sind in diesen Bereichen zum Beispiel verklebt, verschweisst oder miteinander dergestalt verschmolzen, dass sie durch molekulare Kräfte und nicht lösbar miteinander verbunden sind. Bevorzugt ist der distale der beiden Bereiche am distalen Ende der röhrenförmigen Katheterwandung und erstreckt sich von dort noch 1 bis 5 mm in proximaler Richtung, wobei sich der röhrenförmige Führungsmantel noch 1 bis 10 mm aus der ersten distalen Öffnung in distaler Richtung frei, d.h. ohne Kontakt mit der röhrenförmigen Katheterwandung, erstreckt.

Bei der vorliegenden Erfindung sind der röhrenförmige Führungsmantel und die röhrenförmige Katheterwandung ausschliesslich in den besagten Bereichen stoffschlüssig miteinander verbunden. Dies bedeutet, dass über einen weiten Teil einer gesamten Längenausdehnung des Aspirationskatheters, während der ein Führungsmantel koaxial zur Katheterwandung verläuft, der Führungsmantel lose innerhalb des ersten Lumens angeordnet ist. Dies kann zum Beispiel bedeuten, dass lediglich zwischen 0,5 und 8 %, insbesondere zwischen 1 und 5 % der gesamten Längenausdehnung des Führungsmantels stoffschlüssig mit der Katheterwandung verbunden sind.

In einer weiteren besonderen Ausführungsform betragen die Bereiche, in denen die röhrenförmige Katheterwandung mit dem röhrenförmigen Führungsmantel stoffschlüssig verbunden ist, jeweils zwischen 0,5 und 5 mm, bevorzugt zwischen 1 und 3 mm.

Bei der vorliegenden Erfindung verläuft der röhrenförmige Führungsmantel über mindestens einen Teil der Längenausdehnung der röhrenförmigen Katheterwandung koaxial mit dieser. Bevorzugt verläuft der Führungsmantel über eine Länge von zwischen 20 und 30 cm koaxial mit der röhrenförmigen Katheterwandung, bis er an einer Seite lateral austritt. Dieser Teil des Aspirationskatheters erstreckt sich vorzugsweise vom distalen Ende des Katheters 20 bis 30 cm in proximale Richtung und bildet mit der zweiten proximalen Öffnung des röhrenförmigen Führungsmantels und der zweiten distalen Öffnung des röhrenförmigen Führungsmantels den "Rapid Exchange"-Port eines erfindungsgemässen Aspirationskatheters. Besonders bevorzugt ist die zweite proximale Öffnung abgeschrägt, das heisst sie erstreckt sich in einem Winkel zur Längsachse des Katheters, der spitz ist, und vorzugsweise zwischen 5 und 45° beträgt.

In einer besonderen Ausführungsform ist der röhrenförmige Führungsmantel im Wesentlichen ohne Spannung mit seinen beiden stoffschlüssigen Verbindungspunkten mit der Katheterwandung verbunden. Im Sinne der vorliegenden Erfindung ist der röhrenförmige Führungsmantel im Wesentlichen spannungsfrei, wenn in einem geraden Zustand des Aspirationskatheters kein Kräftezug zwischen den beiden Verbindungspunkten des röhrenförmigen Führungsmantels besteht.

In einer besonderen Ausführungsform ist der röhrenförmige Führungsmantel über einen wesentlichen Anteil des Teils, der Längenausdehnung der koaxial mit der röhrenförmigen Katheterwandung verläuft, nicht mit dieser stoffschlüssig verbunden.

Bei der vorliegenden Erfindung ragt der röhrenförmige Führungsmantel an der ersten distalen Öffnung aus dem ersten Lumen hinaus, bevorzugt um eine Länge von zwischen 0.5 und 5 mm. Bevorzugt bildet der Führungsmantel mit seiner zweiten distalen Öffnung das distale Ende des gesamten Aspirationskatheters. Im Betrieb dient die zweite distale Öffnung dazu, den Thrombus oder Embolus, den es abzusaugen gilt, zu durchstossen.

In einer besonderen Ausführungsform ist die erste distale Öffnung bezüglich des Durchmessers der röhrenförmigen Katheterwandung abgeschrägt. In einem konkreten Beispiel bedeutet dies, dass die erste distale Öffnung mit einer Längsachse des Aspirationskatheters einen Winkel von zwischen vorzugsweise 35 und 85°, besonders bevorzugt 45 bis 70° definiert. Bevorzugt ist die erste distale Öffnung dergestalt abgeschrägt, dass der gesamte Öffnungsdurchmesser im Wesentlichen ein Oval beschreibt.

In einer besonderen Ausführungsform ist die Spitze der röhrenförmigen Katheterwandung, insbesondere das distale Ende des besagten Öffnungsovals, abgerundet, sodass keine spitze oder scharfe Kante besteht.

In einer bevorzugten Ausführungsform ist ein distales Ende des Aspirationskatheters mit mindestens einer radioopaken Markierung versehen. Bevorzugt weist der Aspirationskatheter eine erste radioopake Markierung an seinem Führungsmantel auf.

In einer besonderen Ausführungsform ist das Ende des röhrenförmigen Führungsmantels, insbesondere der Teil, der aus der Öffnung hinausragt, mit einem radioopaken Ring versehen. Durch diesen Ring lässt sich die Lage der distalen Katheterspitze im Röntgengerät feststellen und im Körper sicher verfolgen.

In einer besonderen Ausführungsform weist der erfindungsgemässe Aspirationskatheter mindestens eine zweite radioopake Markierung auf. Bevorzugt wird die zweite radioopake Markierung dergestalt am Aspirationskatheter angebracht, dass die relative Lage und Ausrichtung im Raum der ersten distalen Öffnung bestimmt werden können. Dazu kann es insbesondere von Vorteil sein, eine zweite oder weitere radioopake Markierung von der ersten radioopaken Markierung beabstandet anzuordnen.

In einer besonderen Ausführungsform ist die zweite oder weitere radioopake Markierung so an der Katheterwandung angebracht, dass eine grundsätzliche Ausrichtung des Katheters bestimmt werden kann. Dazu wird die zweite oder weitere radioopake Markierung flächig ausgestaltet, d.h, die zweite oder weitere radioopake Markierung weist eine Flächenausdehnung auf.

Somit betrifft die vorliegende Erfindung in einem besonderen Aspekt einen Aspirationskatheter mit einer ersten radioopaken Markierung an seinem distalen Ende, bevorzugt an einem Führungsmantel, welcher aus einer ersten distalen Öffnung einer röhrenförmigen Katheterwandung hinausragt, und einer zweiten radioopaken Markierung, welche von der ersten beabstandet an einer röhrenförmigen Katheterwandung angebracht ist. In einer besonderen Ausführungsform dieses Aspekts ist die zweite oder weitere radioopake Markierung als Pflaster oder Patch auf der gleichen Seite der röhrenförmigen Katheterwandung, mit der der röhrenförmige Führungsmantel stoffschlüssig verbunden ist, angebracht. Bevorzugt ist diese zweite oder weitere radioopake Markierung zurückversetzt, besonders bevorzugt um mindestens 5 mm, weiter besonders bevorzugt um zwischen 5 und 45 mm von der ersten radioopaken Markierung beabstandet.

In einer weiteren besonderen Ausführungsform dieses Aspekts hat die zweite oder weitere radioopake Markierung eine Flächenausdehnung, welche eine lange und eine kurze Kante aufweist. In einer besonderen Ausführungsform ist diese zweite oder weitere radioopake Markierung rechteckig ausgestaltet.

In einer besonderen Ausführungsform ist die zweite radioopake Markierung dergestalt auf der Aussenseite der röhrenförmigen Katheterwandung angebracht, dass ihre Längskante sich mit der Längsachse des Aspirationskatheters kreuzt, vorzugsweise in einem rechten Winkel kreuzt. Besonders bevorzugt erstreckt sich diese zweite oder weitere radioopake Markierung über einen Bereich des Umfangs der röhrenförmigen Katheterwandung. Besonders bevorzugt entspricht dieser Bereich des Umfangs der röhrenförmigen Katheterwandung der Längenausdehnung der zweiten oder weiteren radioopaken Markierung.

In einer besonderen Ausführungsform beschreibt diese Längenausdehnung zwischen 5 und 55, bevorzugt zwischen 10 und 35 % des Umfangs der röhrenförmigen Katheterwandung.

In einer weiteren Ausführungsform dieses Aspekts der vorliegenden Erfindung ist eine zweite oder weitere radioopake Markierung auf der gegenüberliegenden Seite der Katheterwandung bezüglich der Seite der Katheterwandung, mit der der röhrenförmige Führungsmantel stoffschlüssig verbunden ist, angebracht. Somit befindet sich diese zweite oder weitere radioopake Markierung auch auf der gegenüberliegenden Seite der Spitze der röhrenförmigen Katheterwandung in einem konkreten Beispiel. Auch in diesem Beispiel kann die zweite oder weitere radioopake Markierung eine Längen- und eine Breitenausdehnung aufweisen.

In einer besonderen Ausführungsform erstreckt sich die Längenausdehnung in Richtung der I<atheterlängsachse, während die Breitenausdehnung sich in einem rechten Winkel zur I<atheterlängsachse erstreckt.

Grundsätzlich ist es einem Fachmann bekannt, radioopake Markierungen für Aspirationskatheter zu verwenden. Auch die Wahl der Materialien ist einem Fachmann bekannt. Geeignet für alle erfindungsgemässen Aspirationskatheter sind radioopake Markierungen, bestehend aus einer Platin-Iridium (Pt-Ir) - Verbindung im Verhältnis 90 zu 10.

Für das distale Ende des röhrenförmigen Führungsmantels wären zum Beispiel Pt-Ir-Ringe mit einem Aussendurchmesser von 0,6 mm und einem Innendurchmesser von 0,5 mm geeignet.

Für die zweiten oder weiteren radioopaken Markierungen an der röhrenförmigen Katheterwandung wären Pt-Ir-Folien mit einer Dicke von mindestens 0,03 mm geeignet.

Mit diesem Aufbau weist der erfindungsgemässe Aspirationskatheter zusätzlich eine ausreichende Radioopazität auf, um vom Arzt im Körper zuverlässig verortet zu werden. Dies ist bei einer gesamten Katheterlänge von ungefähr 1,5 m durchaus relevant, insbesondere wenn anhand der Drehbewegung ein verbessertes Absaugen von Thromben und/oder Embolien ermöglicht werden soll. Durch den Aufbau des oben genannten Aspekts der vorliegenden Erfindung kann der Arzt zudem bei abgeschrägten Öffnungen der Aspirationslumina stets die Ausrichtung der Aspirationsöffnung erkennen und auch den Effekt der Drehbewegung überwachen.

In einer besonderen Ausführungsform umfasst die Gesamtlänge des erfindungsgemässen Aspirationskatheters 1,5 m.

In einer weiteren besonderen Ausführungsform ist die röhrenförmige Katheterwandung ganz oder teilweise beschichtet. Bevorzugt weist die röhrenförmige Katheterwandung an ihrer Aussenseite auf ihrer gesamten Länge oder auf einem Teil ihrer Länge eine hydrophile Beschichtung auf.

In einer besonderen Ausführungsform ist das zweite Lumen so ausgestaltet, dass es zur Aufnahme eines Führungsdrahtes geeignet ist.

In einer besonderen Ausführungsform ist der erfindungsgemässe Aspirationskatheter so ausgelegt, dass wenn der Aspirationskatheter eine Kurve beschreibt, der röhrenförmige Führungsmantel in seinem Inneren sich stets am engeren Radius der Kurve anschmiegt.

In einer besonderen Ausführungsform bestehen sowohl die röhrenförmige Katheterwandung als auch der röhrenförmige Führungsmantel aus einem Kunststoff, besonders bevorzugt aus einem Polyamid.

In einer besonderen Ausführungsform sind die röhrenförmige Katheterwandung und der röhrenförmige Führungsmantel unterschiedlich eingefärbt. Bevorzugt ist die röhrenförmige Katheterwandung transparent oder semi-transparent.

In einer bevorzugten Ausführungsform weist der erfindungsgemässe Aspirationskatheter einen Aussendurchmesser von zwischen 1,4 und 2 mm, vorzugsweise von zwischen 1,65 und 1,7 mm, besonders bevorzugt von 1,69 mm auf.

In einer bevorzugten Ausführungsform weist der röhrenförmige Führungsmantel einen Durchmesser von zwischen 0,4 und 0,6 mm, bevorzugt von zwischen 0,5 und 0,59 mm, besonders bevorzugt von 0,55 mm auf.

In einer weiteren besonderen Ausführungsform weist der Aspirationskatheter eine Länge von ca. 130 cm auf.

In einer besonderen Ausführungsform bestehen der röhrenförmige Führungsmantel und die röhrenförmige Katheterwandung des erfindungsgemässen Aspirationskatheters aus einem PEEK (einem Polyether-ether-keton).

Ein weiterer Aspekt der vorliegenden Erfindung betrifft einen Komponentensatz zur Aspiration von Material aus Blutgefässen. Im Sinne der vorliegenden Erfindung ist ein Komponentensatz eine Reihe von Vorrichtungen, die als Einheit vertrieben werden können und zusammen im besonderen Fall synergistisch für den bestimmungsgemässen Zweck verwendet werden können. Bei der vorliegenden Erfindung umfasst der Komponentensatz den erfindungsgemäßen Aspirationskatheter.

Der Komponentensatz umfasst weiter mindestens eine Saugvorrichtung zur Erzeugung eines Unterdrucks in einem ersten Lumen, einem Aspirationslumen, des Aspirationskatheters. Der Komponentensatz umfasst weiterhin mindestens einen Absperrhahn zur Verbindung des Aspirationskatheters mit der Saugvorrichtung. Im Sinne der vorliegenden Erfindung ist ein Absperrhahn eine Vorrichtung, die es grundsätzlich ermöglich eine Fluidverbindung zwischen zwei Lumina von einem geschlossenen in einen offenen Zustand zu überführen und umgekehrt. In seiner einfachsten Ausführungsform umfasst der Absperrhahn zwei Öffnungen, welche je mit einem Lumen verbunden werden können und einen I<olben mit einer Bohrung welcher innerhalb des Absperrhahns dergestalt rotiert werden kann, dass die Bohrung eine Fluidverbindung zwischen den zwei Öffnungen ermöglich, oder der Kolben eben diese blockiert. Bevorzugt handelt es sich beim Absperrhahn um einen Einweg-Absperrhahn.

Im Zusammenspiel würde von distal zu proximal eine Absauganordnung zur Aspiration von Material aus Blutgefässen zusammengesetzt werden können mit einem Aspirationskatheter, einem Absperrhahn und einer Saugvorrichtung.

In einer besonderen Ausführungsform handelt es sich bei der Saugvorrichtung um eine Spritze. Geeignete Spritzen sind im medizinischen Fachhandel erhältlich und weisen Volumen von zwischen zum Beispiel 1 und 50 ml auf. Konventionelle, handgetriebene Spitzen weisen einen Kolben auf, durch dessen Rückzug aus dem Spritzenhohlraum ein Unterdruck entsteht und somit eine Ansaugwirkung erzeugt werden kann. Umgekehrt kann durch Hineinschieben des Kolbens in den Spritzenhohlraum ein Inhalt der Spritze durch die Spritzenöffnung injiziert werden. Alternativ zu einer manuell bedienbaren Spritze ist es auch möglich, den erfindungsgemässen Komponentensatz mit einer automatischen Saugvorrichtung zu betreiben. Gemeinsam ist allen möglichen Saugvorrichtungen, dass sie ein Vakuum erzeugen, und dieses mehr oder weniger kontrolliert dazu einsetzen, einen Unterdruck in Downstream gelagerte Elemente übertragen.

In einer besonderen Ausführungsform weist der Komponentensatz eine Codierung auf. Bevorzugt weist der Komponentensatz eine Farbcodierung auf. Durch eine solche Codierung sind alle Elemente bezüglich ihrer Kompatibilität gekennzeichnet.

Im medizinischen Praxisalltag wird als Mass für den Aussendurchmesser von Kathetern die Einheit Charrière verwendet. Die Einheit Charrière wird auch in French (Fr) angegeben. Ein French entspricht dabei einem Drittel Millimeter. Es ist üblich, medizinische Katheter anhand ihres Aussendurchmessers in French zu identifizieren.

In einer besonderen Ausführungsform ist der Komponentensatz ausgelegt für Katheter in der Grössenordnung von zwischen 6 und 7 Fr. Gemeinhin ist es üblich, 6 Fr mit einer Farbkennzeichnung in Orange und 7 Fr mit einer Farbkennzeichnung in Grün zu markieren. Dies erleichtert dem medizinischen Personal, verwandtes Besteck und die entsprechend richtigen Nadeln und Stilette zu identifizieren und anzuwenden.

In einer besonderen Ausführungsform umfasst der Komponentensatz weiter einen Führungskatheter zur Aufnahme des Aspirationskatheters in die Blutgefässe. In der Praxis wird nach der Punktion der Blutgefässe zunächst ein Führungskatheter gesetzt, durch welchen der Führungsdraht eingeführt wird. Später wird auch der Aspirationskatheter entlang des Führungsdrahtes durch diesen Führungskatheter in das Gefäss geschoben.

In einer besonderen Ausführungsform der vorliegenden Erfindung weist der Absperrhahn dieselbe Codierung auf wie die Saugvorrichtung.

Der Absperrhahn, insbesondere der Einwegabsperrhahn, wird vorzugsweise über Luer-Verbindungen mit den jeweiligen Katheterbestandteilen und/oder der Saugvorrichtung verbunden. Dazu ist es entscheidend, dass der Absperrhahn für das entsprechende Aussendurchmessersystem geeignet ist. Geeignete Absperrhähne können somit in den entsprechenden Farben des für sie anwendbaren Durchmessers gekennzeichnet werden.

In einer besonderen Ausführungsform werden Absperrhähne für Komponentensätze mit einem 6-Fr-Aussendurchmesser orange gekennzeichnet, während Absperrhähne für einen Komponentensatz mit 7-Fr-Aussendurchmesser grün gekennzeichnet werden.

In einer weiteren besonderen Ausführungsform des erfindungsgemässen Komponentensatzes weist dieser ein weiteres flexibles Verbindungselement auf. Bevorzugt ist das Verbindungselement ausgestaltet zur Verbindung des Aspirationskatheters mit dem Absperrhahn. Im Sinne der vorliegenden Erfindung sei als flexibel zu verstehen, dass es eine elastische Fähigkeit aufweist. In einem besonderen Beispiel besteht das flexible Verbindungselement aus einem Kunststoff, ausgewählt aus der Gruppe der Kunststoffe mit einer Shore-A-Härte von ungefähr 80. Besonders bevorzugt handelt es sich um einen Kunststoff, ausgewählt aus der Gruppe, bestehend aus: Polyurethane, Silikone und PVC.

Im Betrieb wird das flexible Verbindungselement mit dem proximalen Ende des Aspirationskatheters verbunden. Das flexible Verbindungselement wird anschliessend mit einem Absperrhahn mit einer Saugvorrichtung verbunden. Im Betrieb wird zunächst zwischen der Saugvorrichtung und dem Absperrhahn ein Vakuum erzeugt. Anschliessend wird der Aspirationskatheter über den Führungskatheter in ein punktiertes Blutgefäss eingeführt. Das flexible Verbindungselement dient dazu als Gelenk und erleichtert das Einführen des Katheters.

Ein weiterer besonderer Aspekt betrifft ein Verfahren zur Aspiration von Materialien aus Blutgefässen. Das Verfahren umfasst zunächst das Bereitstellen eines Aspirationskatheters, insbesondere eines eingangs beschriebenen erfindungsgemässen Aspirationskatheters. Mittels eines Stiletts oder einer Nadel wird das Blutgefäss zur Einfuhr eines Führungsdrahtes in das Blutgefäss punktiert. Anschliessend wird ein Führungsdraht im Blutgefäss platziert. Der Aspirationskatheter wird nun mithilfe des Führungsdrahtes eingeführt und das abzusaugende Material, wie zum Beispiel den zu aspirierenden Thrombus oder Embolus, wird mit der Spitze des Aspirationskatheters durchstossen. Der Aspirationskatheter wird anschliessend unter Vollzug einer Drehbewegung um die Längsachse des Aspirationskatheters zurückgezogen und dabei gleichzeitig durch Anbringen eines Vakuums aspiriert.

Bei der Vorbereitung des Katheters kann auch zunächst ein Führungskatheter in das Blutgefäss eingeführt werden. Dieser Führungskatheter erleichtert die Einfuhr des Führungsdrahtes und aller zukünftigen Katheterwechsel.

Beim Einsatz eines erfindungsgemässen Aspirationskatheters wird die Schlag- und Twist-Bewegung des Katheters beim Rotieren um die eigene Längsachse gemindert. Dadurch kann einerseits genauer abgesogen werden und andererseits wird/werden ein unkontrolliertes Schlagen und allfälliges Ansaugen der Gefässinnenwand gehindert.

Für einen Fachmann ist es selbstverständlich, dass sämtliche, sich nicht gegenseitig ausschliessende Ausführungsformen, die oben geschildert wurden, in einer Ausgestaltung des erfindungsgemässen Aspirationskatheters oder Komponentensatzes in beliebiger Kombination verwirklicht werden können.

Grundsätzlich ist der erfindungsgemässe Aspirationskatheter für Venen und/oder Arterien geeignet. Aber auch Anwendungen in anderen Gefässen und Körperhohlräumen sind durchaus denkbar und können von einem Fachmann aus dem erfindungsgemässen Offenbarungsgehalt geschlossen werden.

Im Folgenden wird die Erfindung anhand von Figuren und konkreter Ausführungsbeispiele näher erläutert, ohne jedoch im Schutzumfang auf diese eingeschränkt zu sein.

### Kurze Beschreibung der Zeichnungen

Die zur Erläuterung der Ausführungsbeispiele verwendeten Zeichnungen zeigen:
- Fig. 1: schematisch das distale Ende mit dem "Rapid Exchange"-Port eines erfindungsgemäßen Aspirationskatheters;
- Fig. 2: schematisch eine alternative Ausführungsform mit eingeführtem Führungsdraht;
- Fig. 3: schematisch das distale Ende eines erfindungsgemässen Aspirationskatheters mit radioopaken Markierungen;
- Fig. 4: schematisch einen erfindungsgemässen Aspirationskatheter in Aussenansicht;
- Figur 4a: die Katheterspitze schematisch in Aufsicht;
- Fig. 4b: Querschnitt in der Schnittebene A-A;
- Fig. 4c: Querschnitt in der Schnittebene A'-A";
- Fig. 4d: Querschnitt in der Schnittebene A"-A";
- Fig. 5: Querschnitt durch einen Aspirationskatheter aus dem Stand der Technik;
- Fig. 6: schematisch ein distales Ende eines erfindungsgemässen Aspirationskatheters mit alternativen radioopaken Markierungen;
- Fig. 7: schematisch einen erfindungsgemässen Komponentensatz, und
- Fig. 8: schematisch die Platzierung eines erfindungsgemässen Aspirationskatheters.

Grundsätzlich sind in den Figuren analoge Elemente mit den gleichen Bezugszeichen versehen.

### Wege zur Ausführung der Erfindung

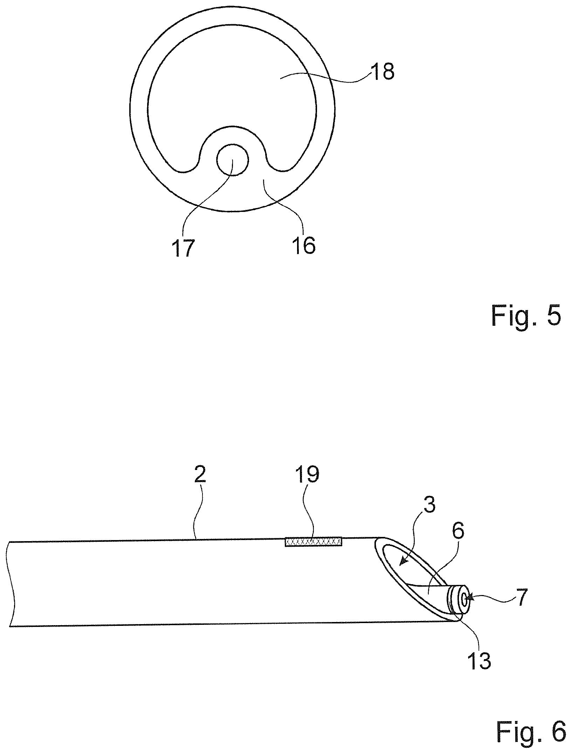

Am Beispiel der Figur 5 sieht man einen Querschnitt durch einen gängigen Aspirationskatheter, wie er im Stand der Technik bekannt ist und Verwendung findet. Die Katheterwandung 16 ist so ausgestaltet, dass sie ein Stück ein Aspirationslumen 18 und ein Führungsdrahtlumen 17 umschliesst. Es ist klar ersichtlich, dass wenn sich ein solcher Querschnitt durch die gesamte Länge des vom Führungsdraht geführten Bereichs erstreckt, eine einseitige Steifigkeit an der Seite der Katheterwandung besteht, an der das Führungsdrahtlumen 17 ausgebildet ist. Wird ein solcher Katheter um die eigene Achse rotiert, so führt er eine Twist- oder Schlagbewegung aus.

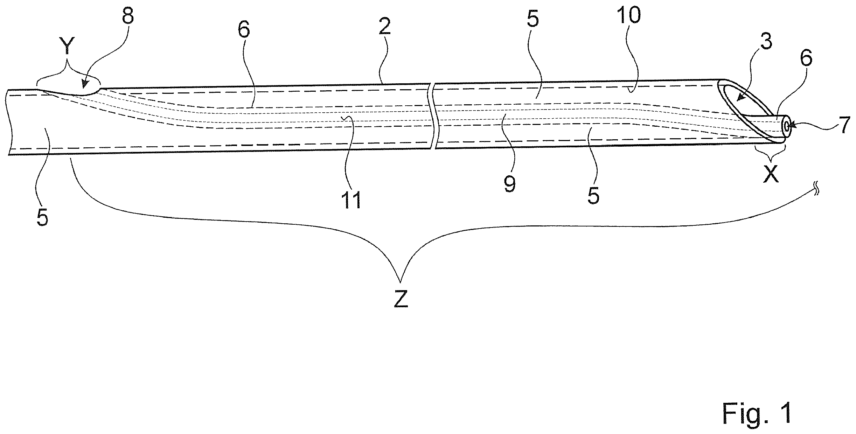

Die Figur 1 zeigt schematisch eine Ausführungsform des erfindungsgemässen Aspirationskatheters. Der dargestellte Bereich Z entspricht in etwa dem geführten Bereich, respektive dem "Rapid Exchange"-Port Z. Der Aspirationskatheter weist eine röhrenförmige Katheterwandung 2 auf, welche in ihrem Inneren ein erstes Lumen 5 definiert. Der Aussendurchmesser der Katheterwandung 2 beträgt in diesem konkreten Beispiel 1,69 mm. Die Wandstärke beträgt ca. 0,075 mm. Die Katheterinnenwand 10 umschliesst das erste Lumen 5, welches an seinem distalen Ende über eine erste distale Öffnung 3 in Fluidverbindung mit einem Gefässinnenraum (nicht gezeigt) gebracht werden kann. Das proximale Ende der röhrenförmigen Katheterwandung 2 ist in diesem Beispiel nicht gezeigt. Innerhalb der röhrenförmigen Katheterwandung 2 erstreckt sich ein röhrenförmiger Führungsmantel 6 mit einem Aussendurchmesser von 0,55 mm. Dieser wiederum definiert über eine Führungsmantelinnenwand 11 ein zweites Lumen 9, welches als Führungsdrahtlumen 9 dient. Am distalen Ende ragt der Führungsmantel 6 zwischen 0,5 und 8 mm aus der röhrenförmigen Katheterwandung 2 hinaus und verfügt über eine zweite distale Öffnung 7.

Das proximale Ende des röhrenförmigen Führungsmantels 6 erstreckt sich lateral durch die röhrenförmige Katheterwandung und bildet dort eine zweite proximale Öffnung 8. Dabei ist in dieser Zeichnung lediglich schematisch die Öffnung auf der gegenüberliegenden Seite der Spitze des Führungsmantels gezeigt. Die laterale Öffnung kann an einer beliebigen Stelle des Umfangs der röhrenförmigen Katheterwandung 2 austreten.

Der röhrenförmige Führungsmantel ist ausschliesslich in den Bereichen X, Y, das heisst in einem Bereich der ersten distalen Öffnung und in einem Bereich der zweiten proximalen Öffnung mit der röhrenförmigen Katheterwandung 2 verbunden. Im vorliegenden Beispiel sind sowohl die röhrenförmige Katheterwandung 2 als auch der röhrenförmige Führungsmantel 6 aus PEEK und miteinander in den Bereichen X, Y stoffschlüssig verbunden, beispielsweise verschweisst. Vorzugsweise besteht nahezu keine oder nur eine vernachlässigbare Spannung an den Schweisspunkten in den Bereichen X, Y, wenn der Katheter gerade und gestreckt ist.

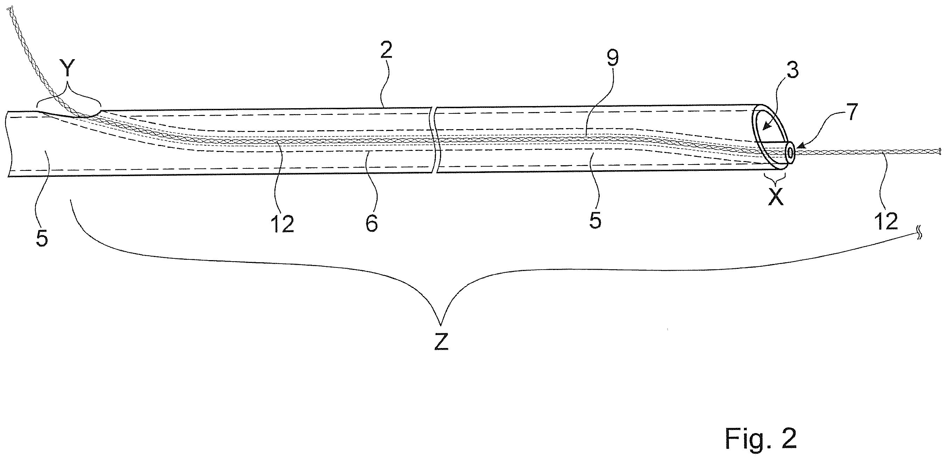

In der Figur 2 wird der gezeigte "Rapid Exchange"-Port Z mit einem eingeführten Führungsdraht 12, welcher sich durch die gesamte Längsachse des röhrenförmigen Führungsmantels 6 erstreckt, gezeigt. Im Betrieb wird der Führungsdraht 12 in die zweite distale Öffnung 7 eingeführt und durch die zweite proximale Öffnung 8 wieder hinausgeführt. Mithilfe dieser Führung wird der Katheter vorgeschoben durch das Blutgefäss bis zur Obstruktion. Die Katheterwandung 2 in diesem Ausführungsbeispiel weist eine erste distale Öffnung 3 auf, die einen weniger spitzen Winkel als die Figur 1 aufweist. Im vorliegenden Beispiel hat die Spitze des Aspirationskatheters einen Winkel von 45°. Auch in diesem Ausführungsbeispiel ist der röhrenförmige Führungsmantel an seiner lateralen Austrittsstelle Y und an einer Kontaktstelle X an der distalen Öffnung 3 mit der röhrenförmigen Katheterwandung 2 stoffschlüssig verbunden. Der röhrenförmige Führungsmantel verläuft somit durch das Lumen 5 der röhrenförmigen Katheterwandung 2.

In den beiden gezeigten Ausführungsbeispielen der Figuren 1 und 2 sind die radioopaken Markierungen nicht gezeigt, können aber selbstredend am distalen Ende des Aspirationskatheters vorgesehen sein.

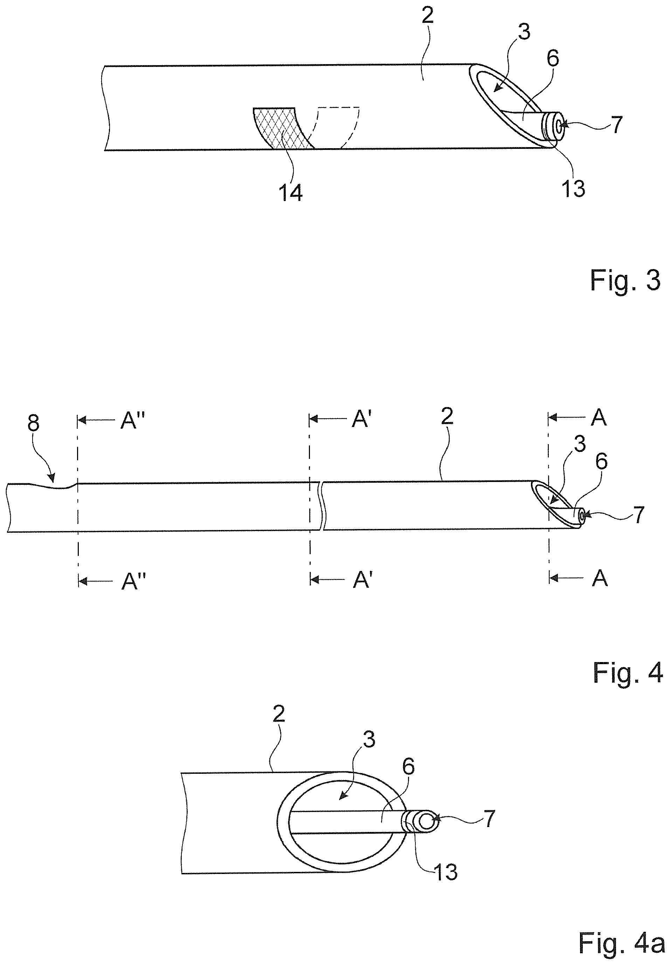

In der Figur 3 wird ein Ausführungsbeispiel mit entsprechend platzierten radioopaken Markierungen 13, 14 schematisch dargestellt. Gezeigt ist das distale Ende eines erfindungsgemässen Aspirationskatheters. Die röhrenförmige Katheterwandung 2 endet mit einer abgeschrägten Aspirationsöffnung 3, durch welche sich der röhrenförmige Führungsmantel 6 mit seiner distalen Öffnung 7 erstreckt. Der röhrenförmige Führungsmantel 6 ist mit einem radioopaken PT-IR-Ring an seinem distalen Ende radioopak markiert. In proximaler Richtung ist, beabstandet von diesem ersten radioopaken Marker 13, eine radioopake Pt-Ir-Folie 14 auf der gleichen Seite wie die Klebestelle des Führungsmantels aussen an der Katheterwandung 2 aufgebracht. Dieser Marker kann geklebt, aufgeschweisst oder aufgelötet sein und verfügt über eine Materialdecke von 0,03 mm. Im gezeigten Beispiel ist die Folie 14 so ausgelegt, dass sie den Umfang der röhrenförmigen Katheterwandung 2 mindestens zur Hälfte umschliesst. Durch die Geometrie dieser radioopaken Folie kann die Ausrichtung des Aspirationskatheters am Röntgengerät sichtbar gemacht werden. Anhand der Öffnung der Folie kann ein behandelnder Chirurg bestimmen, in welche Richtung sich die ovale Aspirationsöffnung 3 öffnet.

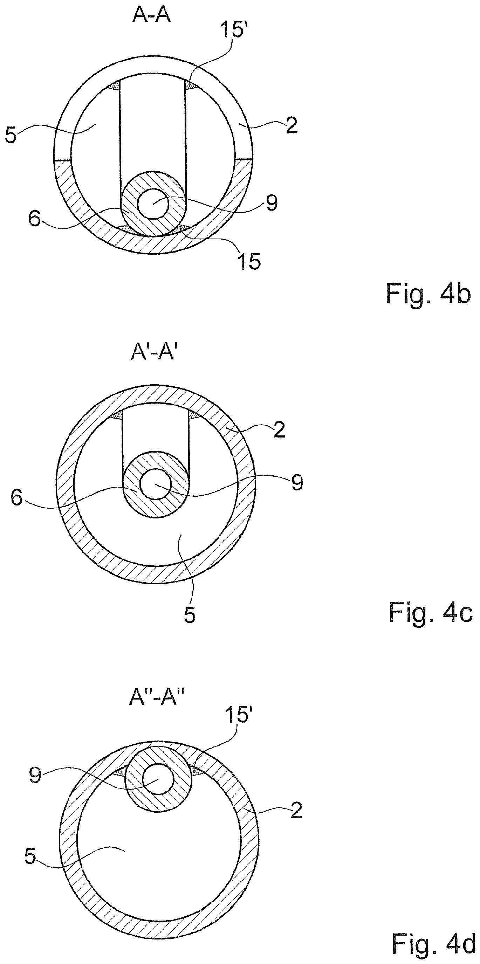

In der Figur 4 wird zur besseren Illustration erneut die erfindungsgemässe Katheterspitze mit der Katheterwandung 2, der ersten distalen Öffnung 3, der zweiten distalen Öffnung 7, dem röhrenförmigen Führungsmantel 6 und der zweiten proximalen Öffnung 8 anhand verschiedener Querschnitte aufgezeigt.

In der Figur 4a ist die ovale, erste distale Öffnung 3 mit dem sich daraus erstreckenden röhrenförmigen Führungsmantel 6 mit der zweiten distalen Öffnung 7 und dem radioopaken Pt-Ir-Ring 13 gezeigt.

Die Figur 4b illustriert den Querschnitt in der Schnittebene A-A der Figur 4 an der Katheterspitze. Der röhrenförmige Führungsmantel 6 ist mittels einer Schweissnaht 15 mit der Katheterwandung 2 stoffschlüssig verbunden und erstreckt sich aus der Schnittebene nach hinten durch das Aspirationslumen 5 bis zu seinem Austritt, wo er erneut stoffschlüssig mit der Katheterwandung über eine Schweissnaht 15' verbunden ist.

Die Figur 4c illustriert den Zwischenraum zwischen den beiden Öffnungen des Führungsmantels in der Schnittebene A'-A'. Der Führungsmantel 6 ist frei im ersten Lumen 5 der röhrenförmigen Katheterwandung 2 angeordnet.

In der Figur 4d ist das proximale Ende des Führungsmantels gezeigt, wo der Führungsmantel 6 mit der röhrenförmigen Katheterwandung 2 erneut stoffschlüssig über eine Schweissnaht 15' verbunden ist.

In der Figur 6 wird eine Alternativ- oder ergänzende Anordnung für die radioopaken Markierungen 13, 19 gezeigt. Neben der breiten Verbindung mit der Figur 3 besprochenen radioopaken Ring 13 am distalen Ende des röhrenförmigen Führungsmantels verfügt dieser Aspirationskatheter über eine weitere rechteckige Pt-Ir-Folie 19, welche auf der gegenüberliegenden Seite, und zwar mit einer Längskante in Längsrichtung des Aspirationskatheters angebrachte radioopake Folie 19. Durch diese Marker ist es einem Chirurgen ebenfalls möglich, Lage und Ausrichtung des Aspirationskatheters zuverlässig zu bestimmen.

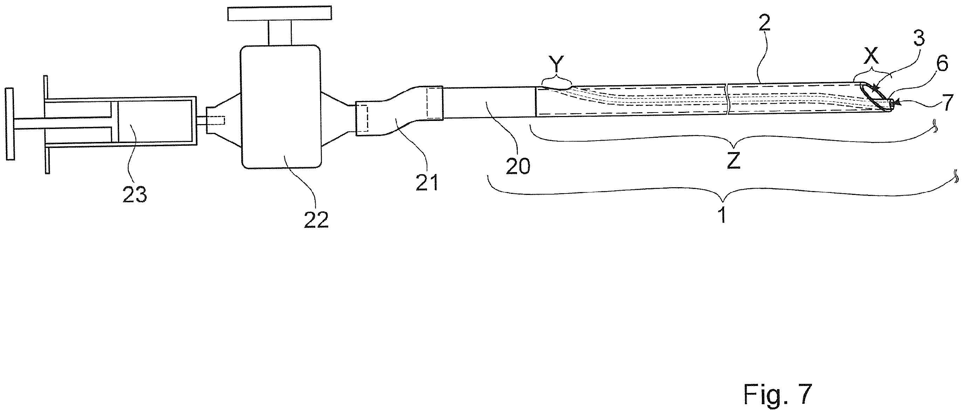

In der Figur 7 wird ein erfindungsgemässer Komponentensatz schematisch und nicht in Grössenrelation gezeigt. Der erfindungsgemässe Aspirationskatheter 1 umfasst einen distalen "Rapid Exchange"-Port Z, der neben einer röhrenförmigen Katheterwandung 2 einen innerhalb des Aspirationslumens 5 bis auf seine Bereiche Y, X frei beweglichen röhrenförmigen Führungsmantel 6 umfasst. Dieser ist über seine Kontaktbereiche X, Y mit der röhrenförmigen Katheterwandung 2 stoffschlüssig verbunden. Der "Rapid Exchange"-Port Z ist ungefähr 20 bis 30 cm lang und endet in Katheterteil 20, welcher weitere 80 bis 100 cm lang ist. Der "Rapid Exchange"-Port Z kann mit diesem Katheterteil 20 stoffschlüssig verbunden sein. Es ist aber auch denkbar, dass die röhrenförmige Katheterwandung 2 mit dem Katheterteil 20 einstückig ist und eine Fortsetzung der röhrenförmigen Katheterwandung darstellt. Insgesamt bildet die gesamte Länge des I<atheterteils 20 mit dem "Rapid Exchange"-Port Z den Aspirationskatheter 1, der an seinem proximalen Ende mittels eines flexiblen PU-Schlauches 21 mit einem Einwegabsperrhahn 22 verbunden ist. Dieser wiederum ist mit einer Spritze 23 verbunden.

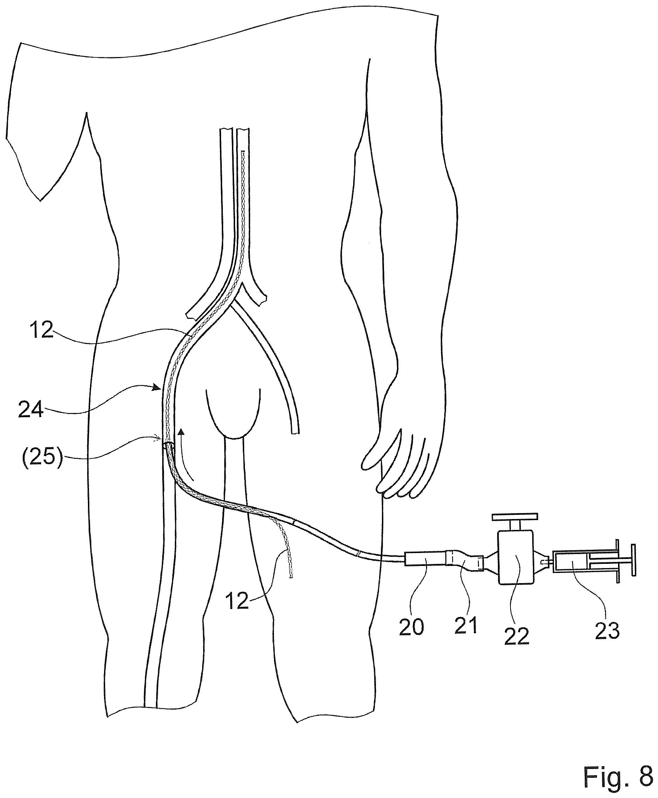

In der Figur 8 ist exemplarisch die Verwendung eines erfindungsgemässen Komponentensatzes mit einem entsprechenden Aspirationskatheter gezeigt. Ein solches Verfahren würde zum Beispiel bei einer perkutanen Thrombusaspiration eingesetzt. dabei wird die Oberschenkelarterie (Arteria femoralis) punktiert und ein Führungsdraht in die Arterie über die Aorta ins Herz geführt.

Zur Platzierung des Führungsdrahtes wird ein Führungskatheter verwendet. Dieser Führungskatheter kann zum Beispiel zu einem 6-Fr- oder einem 7-Fr-Kathetersystem gehören. Durch die Wahl des Führungskatheters bestimmt sich die Auswahl der entsprechenden nachfolgenden Komponenten, wobei Absperrhahn 22 und Spritze 23 sowie der zu verwendete Aspirationskatheter ebenfalls über eine entsprechende Farbcodierung verfügen können.

Wird der Führungskatheter wieder entfernt, kann der Aspirationskatheter über die Punktion 25 durch die Femoralarterie 24 zum Obstruktionsherd (nicht gezeigt) geführt werden. Mit dem Aspirationskatheter wird der Obstruktionsherd durchstossen und anschliessend, während der Aspirationskatheter zurückgezogen wird, aspiriert.

Dabei wird gleichzeitig eine Drehbewegung des Aspirationskatheters vollführt, durch welchen die Absaugung möglichst gleichmässig stattfindet. Grundsätzlich wird bereits vor Einfuhr des Aspirationskatheters mittels der Spritze ein Unterdruck bei geschlossenem Absperrhahn erzeugt. Wird der Absperrhahn geöffnet, so erzeugt der Unterdruck einen Ansaugeffel<t durch das Lumen des Katheters bis zur distalen Öffnung des Aspirationskatheters. Zu Beginn der Prozedur kann es ratsam sein, den Katheter oder die Spritze mit physiologischer Kochsalzlösung auszuspülen.

Der erfindungsgemässe Aspirationskatheter sowie der Komponentensatz sind selbstverständlich auch für Thromben und Embolien an anderen Orten des Körpers einsetzbar.

## Patentansprüche

1. Aspirationskatheter zur Aspiration von Material aus Blutgefässen, umfassend
eine röhrenförmige Katheterwandung (2) mit einer ersten distalen Öffnung (3) und einer ersten proximalen Öffnung (4), welche durch ein erstes Lumen (5) verbunden sind;
ein röhrenförmiger Führungsmantel (6) mit einer zweiten distalen Öffnung (7) und einer zweiten proximalen Öffnung (8), welche durch ein zweites Lumen (9) verbunden sind, und wobei
der röhrenförmige Führungsmantel (6) innerhalb des ersten Lumens (5) angeordnet ist, und wobei
die zweite proximale Öffnung (8) sich lateral durch die röhrenförmigen Katheterwandung (2) erstreckt, und das zweite Lumen (9) koaxial zum ersten Lumen (5) verläuft, und wobei
der röhrenförmige Führungsmantel (6) in einem Bereich (X) der ersten distalen Öffnung (3) und in einem Bereich (Y) der zweiten proximalen Öffnung (8) mit der röhrenförmigen Katheterwandung (2) verbunden ist, wobei der röhrenförmige Führungsmantel (6) und die röhrenförmige Katheterwandung (2) ausschliesslich in den besagten Bereichen stoffschlüssig miteinander verbunden sind, so dass über einen weiten Teil einer gesamten Längenausdehnung des Aspirationskatheters, während der der Führungsmantel in der Katheterwandung verläuft, der Führungsmantel lose innerhalb des ersten Lumens angeordnet ist,
**dadurch gekennzeichnet, dass**
der röhrenförmige Führungsmantel (6) an der ersten distalen Öffnung (3) aus dem ersten Lumen (5) hinausragt.

2. Aspirationskatheter gemäss Anspruch 1, wobei der röhrenförmige Führungsmantel (6) über 75% bis 99% der Länge dieses Teils nicht stoffschlüssig mit der röhrenförmigen Katheterwandung (2) verbunden ist.

3. Aspirationskatheter gemäss einem der Ansprüche 1 bis 2, wobei die zweite distale Öffnung (7) das distale Ende des Aspirationskatheters bildet.

4. Aspirationskatheter gemäss einem der Ansprüche 1 bis 3, wobei die erste distale Öffnung (3) bezüglich des Durchmessers der röhrenförmigen Katheterwandung (2) abgeschrägt ist.

5. Aspirationskatheter gemäss einem der Ansprüche 1 bis 4 wobei der Aspirationskatheter mindestens eine erste radioopake Markierung (13) an seinem distalen Ende aufweist.

6. Aspirationskatheter gemäss Anspruch 5, wobei der Aspirationskatheter mindestens eine weitere radioopake Markierung (14, 19) aufweist, insbesondere mindestens eine weitere radioopake Markierung (14. 19) aufweist, die von der ersten radioopaken Markierung (13) beabstandet ist.

7. Aspirationskatheter gemäss einem der Ansprüche 1 bis 6, wobei der Aspirationskatheter mindestens eine erste radioopake Markierung (13) und mindesten eine zweite radioopake Markierung (14, 19) umfasst und wobei die zweite radioopake Markierung (14, 19) eine Flächenausdehnung aufweist, durch welche die Ausrichtung des ersten distalen Öffnung (3) anhand eines Röntgenbildes bestimmt werden kann.

8. Komponentensatz zur Aspiration von Material aus Blutgefässen, umfassend die folgenden Elemente:
mindestens einen Aspirationskatheter gemäss einem der Ansprüche 1 bis 7;
mindestens eine Saugvorrichtung (23) zur Erzeugung eines Unterdruckes im ersten Lumen (5) des Aspirationskatheters;
mindestens einen Absperrhahn (22) zur Verbindung des Aspirationskatheters mit der Sauvorrichtung (23).

9. Komponentensatz gemäss Anspruch 8, wobei der Komponentensatz eine I<odierung, insbesondere eine Farbkodierung, aufweist, welche alle besagten Elemente entsprechend ihrem Aussendurchmesser und ihrer Kompatibilität kennzeichnet.

10. Komponentensatz gemäss Anspruch 9, wobei der Absperrhahn dieselbe Kodierung aufweist wie die Saugvorrichtung (23), insbesondere eine Farbkodierung entsprechend seinem Charrière-Durchmesser aufweist.

11. Komponentensatz gemäss einem der Ansprüche 8 bis 10, wobei der Komponentensatz weiter ein flexibles Verbindungselement (21) umfasst, insbesondere ein flexibles Verbindungselement (21) zur Verbindung des Aspirationskatheters mit dem Absperrhahn (22) umfasst.

12. Komponentensatz gemäss Anspruch 11, wobei das flexible Verbindungselement aus einem Material besteht welches eine Shore-A Härte von ungefähr 80 aufweist.

## Claims

1. Aspiration catheter for aspirating material from blood vessels, comprising
a tubular catheter wall (2) with a first distal opening (3) and a first proximal opening (4) connected by a first lumen (5);
a tubular guide sleeve (6) with a second distal opening (7) and a second proximal opening (8) which are connected by a second lumen (9) and wherein
the tubular guide sleeve (6) is arranged inside the first lumen (5), and wherein
the second proximal opening (8) extends laterally through the tubular catheter wall (2), and the second lumen (9) is coaxial with the first lumen (5), and wherein
the tubular guide sleeve (6) is connected to the tubular catheter wall (2) in a region (X) of the first distal opening (3) and in a region (Y) of the second proximal opening (8), the tubular guide sleeve (6) and the tubular catheter wall (2) being bonded to each other by a material bond exclusively in said areas, so that over a large part of the entire length of the aspiration catheter, during which the guide sleeve runs in the catheter wall, the guide sleeve is loosely arranged within the first lumen,
**characterized in that**
the tubular guide sleeve (6) protrudes from the first lumen (5) at the first distal opening (3).

2. Aspiration catheter according to claim 1, wherein the tubular guide sleeve (6) is not materially bonded to the tubular catheter wall (2) over 75% to 99% of the length of this part.

3. Aspiration catheter according to one of claims 1 to 2, wherein the second distal opening (7) forms the distal end of the aspiration catheter.

4. Aspiration catheter according to one of claims 1 to 3, wherein the first distal opening (3) is chamfered with respect to the diameter of the tubular catheter wall (2).

5. Aspiration catheter according to one of claims 1 to 4, wherein the aspiration catheter has at least a first radio-opaque marking (13) at its distal end.

6. Aspiration catheter according to claim 5, wherein the aspiration catheter comprises at least one further radio-opaque marking (14, 19), in particular at least one further radio-opaque marking (14, 19) which is spaced apart from the first radio-opaque marking (13).

7. An aspiration catheter according to any one of claims 1 to 6, wherein the aspiration catheter comprises at least a first radio-opaque marking (13) and at least a second radio-opaque marking (14, 19), and wherein the second radio-opaque marking (14, 19) comprises a surface area by which the orientation of the first distal opening (3) can be determined from an X-ray image.

8. A set of components for aspirating material from blood vessels, comprising the following elements:
at least one aspiration catheter according to one of claims 1 to 7;
at least one suction device (23) for generating a vacuum in the first lumen (5) of the aspiration catheter;
at least one stopcock (22) for connecting the aspiration catheter to the suction device (23).

9. A set of components according to claim 8, wherein the set of components comprises a coding, in particular a color coding, which identifies all said elements according to their outer diameter and their compatibility.

10. A set of components according to claim 9, wherein the stopcock comprises the same coding as the suction device (23), in particular a color coding corresponding to its Charrière diameter.

11. A component set according to one of claims 8 to 10, wherein the component set further comprises a flexible connecting element (21), in particular a flexible connecting element (21) for connecting the aspiration catheter to the stopcock (22).

12. A component set according to claim 11, wherein the flexible connecting element is made of a material which comprises a Shore A hardness of about 80.

## Revendications

1. Cathéter d'aspiration pour l'aspiration de matière hors de vaisseaux sanguins, comprenant
une paroi de cathéter de forme tubulaire (2) dotée d'une première ouverture distale (3) et d'une première ouverture proximale (4) qui sont connectées par un premier lumen (5) ;
une chemise de guidage de forme tubulaire (6) dotée d'une seconde ouverture distale (7) et d'une seconde ouverture proximale (8) qui sont connectées par un second lumen (9), et
la chemise de guidage de forme tubulaire (6) étant disposée à l'intérieur du premier lumen (5), et
la seconde ouverture proximale (8) s'étendant latéralement à travers la paroi de cathéter de forme tubulaire (2), et le second lumen (9) s'étendant coaxialement par rapport au premier lumen (5), et
la chemise de guidage de forme tubulaire (6) étant connectée, dans une zone (X) de la première ouverture proximale (8) et dans une zone (Y) de la seconde ouverture proximale (8), à la paroi de cathéter de forme tubulaire (2), la chemise de guidage de forme tubulaire (6) et la paroi de cathéter de forme tubulaire (2) étant connectées exclusivement l'une à l'autre par correspondance de matière dans lesdites zones, de sorte que, par l'intermédiaire d'une large partie d'une extension longitudinale totale du cathéter d'aspiration, sur laquelle la chemise de guidage s'étend dans la paroi de cathéter, la chemise de guidage est disposée de manière lâche à l'intérieur du premier lumen,
**caractérisé en ce que**
la chemise de guidage de forme tubulaire (6) déborde du premier lumen (5) au niveau de la première ouverture distale (3).

2. Cathéter d'aspiration selon la revendication 1, dans lequel la chemise de guidage de forme tubulaire (6) n'est pas connectée correspondance de matière, sur 75 % à 99 % de la longueur de cette partie, à la paroi de cathéter de forme tubulaire (2).

3. Cathéter d'aspiration selon une des revendications 1 à 2, dans lequel la seconde ouverture distale (7) constitue l'extrémité distale du cathéter d'aspiration.

4. Cathéter d'aspiration selon une des revendications 1 à 3, dans lequel la première ouverture distale (3) est biseautée par rapport au diamètre de la paroi de cathéter de forme tubulaire (2).

5. Cathéter d'aspiration selon une des revendications 1 à 4, dans lequel le cathéter d'aspiration présente au moins un marquage radio-opaque (13) à son extrémité distale.

6. Cathéter d'aspiration selon la revendication 5, dans lequel le cathéter d'aspiration présente au moins un autre marquage radio-opaque (14, 19), en particulier au moins un autre marquage radio-opaque (14, 19) qui est espacé du premier marquage radio-opaque (13).

7. Cathéter d'aspiration selon une des revendications 1 à 6, dans lequel le cathéter d'aspiration présente au moins un premier marquage radio-opaque (13) et au moins un second marquage radio-opaque (14, 19) et le second marquage radio-opaque (14, 19) présente une extension superficielle grâce à laquelle l'orientation de la première ouverture distale (3) peut être définie à partir d'une image radiographique.

8. Ensemble de composants pour l'aspiration de matériau hors de vaisseaux sanguins, comprenant les éléments suivants :
au moins un cathéter d'aspiration selon une des revendications 1 à 7 ;
au moins un dispositif d'aspiration (23) pour générer une dépression dans le premier lumen (5) du cathéter d'aspiration ;
au moins un robinet de blocage (22) pour connecter le cathéter d'aspiration au dispositif d'aspiration (23).

9. Ensemble de composants selon la revendication 8, l'ensemble de composants présentant un codage, en particulier un codage couleur qui identifie tous lesdits éléments en fonction de leur diamètre extérieur et de leur compatibilité.

10. Ensemble de composants selon la revendication 9, dans lequel le robinet de blocage présente le même codage que le dispositif d'aspiration (23), en particulier un codage couleur en fonction de son diamètre Charrière.

11. Ensemble de composants selon une des revendications 8 à 10, dans lequel l'ensemble de composants comprend en outre un autre élément de connexion flexible (21), en particulier un élément de connexion flexible (21) pour connecter le cathéter d'aspiration au robinet de blocage (22).

12. Ensemble de composants selon la revendication 11, dans lequel l'élément de connexion est composé d'un matériau qui présente une dureté Shore A d'environ 80.
